# EUROPÄISCHE PATENTSCHRIFT

(11) **EP 2 991 973 B1**
(45) Veröffentlichungstag und Bekanntmachung des Hinweises auf die Patenterteilung: **18.04.2018**
(21) Anmeldenummer: 14719764.4
(22) Anmeldetag: 28.04.2014
(51) Int. Cl.: C07D 309/10, C11B 9/00

(54) **VERFAHREN ZUR HERSTELLUNG VON 2-SUBSTITUIERTEN 4-HYDROXY-4-METHYL-TETRAHYDROPYRANEN MIT RÜCKFÜHRUNG**
PROCESS FOR THE PREPARATION OF 2-SUBSTITUTED 4-HYDROXY-4-METHYL-TETRAHYDROPYRANS WITH RECIRCULATION
PROCÉDÉ DE PRODUCTION DE 4-HYDROXY-4-MÉTHYL-TÉTRAHYDROPYRANES 2-SUBSTITUÉS AVEC RECYCLAGE

(30) Priorität: 29.04.2013 EP 13165778
(43) Veröffentlichungstag der Anmeldung: 09.03.2016
(73) Patentinhaber: BASF SE, 67056 Ludwigshafen am Rhein (DE)
(72) Erfinder: STORK, Timon, 68163 Mannheim (DE); BECK, Karl, 76684 Östringen (DE); GRALLA, Gabriele, 68161 Mannheim (DE); BEY, Oliver, 67150 Niederkirchen (DE); EBEL, Klaus, 68542 Heddesheim (DE)
(74) Vertreter: BASF IP Association
(86) Internationale Anmeldenummer: PCT/EP2014/058536
(87) Internationale Veröffentlichungsnummer: WO 2014/177484

(56) Entgegenhaltungen:
- WO-A1-2010/133473
- WO-A1-2011/154330
- US-A1- 2011 295 024
- GEVORKN A A ET AL: "Mechanism of cycloalkylation of allylcarbinols with aldehydes and ketones", CHEMISTRY OF HETEROCYCLIC COMPOUNDS, SPRINGER NEW YORK LLC, US, Nr. 12, 1. Januar 1982 (1982-01-01), Seiten 1240-1242, XP002597060, ISSN: 0009-3122
- ALEXANDRA MACEDO ET AL: "Solvent-free catalyzed synthesis of tetrahydropyran odorants: the role of SiO2.p-TSA catalyst on the Prins-cyclization reaction", JOURNAL OF THE BRAZILIAN CHEMICAL SOCIETY, SAO PAULO, BR, Bd. 21, Nr. 8, 4. Mai 2010 (2010-05-04), Seiten 1563-1571, XP002661261, ISSN: 0103-5053, DOI: 10.1590/S0103-50532010000800023

## Beschreibung

### HINTERGRUND DER ERFINDUNG

Die vorliegende Erfindung betrifft ein Verfahren zur Herstellung von 2-substituierten 4-Hydroxy-4-methyl-tetrahydropyranen.

### STAND DER TECHNIK

2-substituierte 4-Hydroxy-4-methyl-tetrahydropyrane sind wertvolle Verbindungen für den Einsatz als Aromachemikalien. So zeichnet sich beispielsweise das cis/trans-Diastereomerengemisch des 2-(2-Methylpropyl)-4-hydroxy-4-methyl-tetrahydropyrans 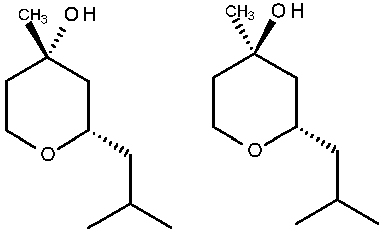 durch einen angenehmen Maiglöckchenduft aus und ist in besonderem Maße zur Verwendung als Aromachemikalie, z. B. zur Herstellung von Riechstoffkompositionen, geeignet.

Die EP 1 493 737 A1 offenbart ein Verfahren zur Herstellung von Gemischen von ethylenisch ungesättigten 4-Methyl- bzw. 4-Methylenpyranen und den entsprechenden 4-Hydroxypyranen durch Umsetzung der entsprechenden Aldehyde mit Isoprenol, wobei die Umsetzung in einem Reaktionssystem initiiert wird, in dem das molare Verhältnis von Aldehyd zu Isoprenol größer als 1 ist, d. h. der Aldehyd im Überschuss eingesetzt wird. Darüber hinaus offenbart das Dokument die anschließende Dehydratisierung der genannten Gemische zu den gewünschten ethylenisch ungesättigten Pyranen. Als geeignete Katalysatoren für den ersten Reaktionsschritt werden Mineralsäuren wie Salzsäure oder Schwefelsäure, bevorzugt jedoch Methansulfonsäure oder p-Toluolsulfonsäure genannt.

EP 1 516 879 A1 offenbart ein Verfahren zur Herstellung von ethylenisch ungesättigten 4-Methyl- bzw. 4-Methylenpyranen durch Umsetzung eines entsprechenden Aldehyds mit Isoprenol unter dehydratisierenden Bedingungen, wobei die Wassermenge im Reaktor bis zu 0,25 Gew.-% beträgt, während der Umsatz der im Unterschuss eingesetzten Ausgangsverbindung weniger als 50 % beträgt. Als hierfür geeignete Katalysatoren werden ebenfalls Mineralsäuren wie Salzsäure oder Schwefelsäure, bevorzugt jedoch Methansulfonsäure oder p-Toluolsulfonsäure, genannt.

Die WO 2010/133473 beschreibt ein Verfahren zur Herstellung von 2-substituierten 4-Hydroxy-4-methyl-tetrahydropyranen der Formel (I) 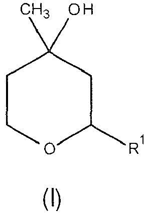 wobei der Rest R¹ für einen geradkettigen oder verzweigten Alkyl- oder Alkenylrest mit 1 bis 12 Kohlenstoffatomen, einen gegebenenfalls Alkyl-substituierten Cycloalkylrest mit insgesamt 3 bis 12 Kohlenstoffatomen oder einen gegebenenfalls Alkyl- und/oder Alkoxy-substituierten Arylrest mit insgesamt 6 bis 12 Kohlenstoffatomen steht, bei dem man Isoprenol (3-Methylbut-3-en-1-ol) mit einem Aldehyd der Formel R¹-CHO umsetzt, wobei man die Umsetzung in Gegenwart von Wasser und in Gegenwart eines stark sauren Kationenaustauschers durchführt.

Die US 2011/295024 A1 beschreibt ein kontinuierliches Verfahren zur Herstellung von 2-substituierten 4-Hydroxy-4-methyl-tetrahydropyranen aus 3-Methylbut-3-en-1-ol und einem Aldehyd in Gegenwart eines sauren Ionentauschers und Wasser.

Die WO 2011/154330 beschreibt ein zur WO 2010/133473 vergleichbares Verfahren, wobei das erhaltene Reaktionsgemisch einer destillativen Aufarbeitung in einer Trennwandkolonne oder in zwei thermisch gekoppelten Destillationskolonnen zugeführt wird.

Die unveröffentlichte europäische Patentanmeldung 12188518.0 beschreibt ein Verfahren zur Herstellung von 2-substituierten 4-Hydroxy-4-methyl-tetrahydropyranen der allgemeinen Formel (I) und von 2-substituierten 4-Methyl-tetrahydropyranen der allgemeinen Formel (II) 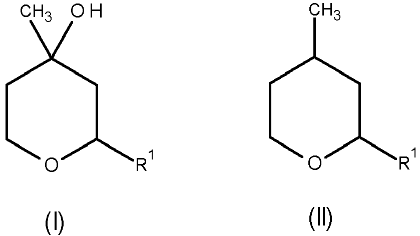 worin
- R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
bei dem man
a) 3-Methylbut-3-en-1-ol der Formel (III) 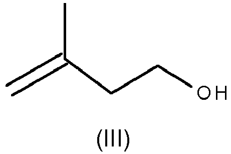 mit einem Aldehyd der Formel (IV)

   R¹-CHO (IV)

   wobei R¹ in der Formel (IV) die zuvor angegebene Bedeutung hat,
   in Gegenwart eines sauren Katalysators umsetzt, wobei ein Reaktionsgemisch erhalten wird, das wenigstens ein 2-substituiertes 4-Hydroxy-4-methyl-tetrahydropyran der allgemeinen Formel (I), wenigstens eine der Verbindungen (V.1), (V.2) oder (V.3) und wenigstens eine Dioxanverbindung (VI) enthält 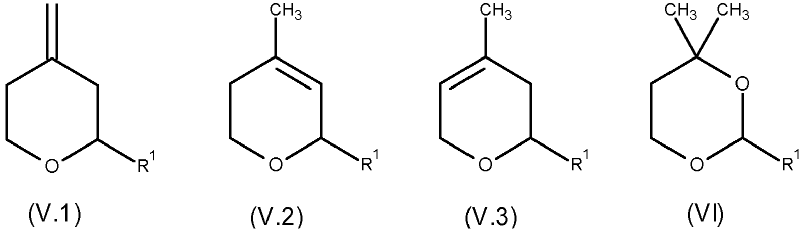 wobei R¹ in der Formel (VI) die zuvor angegebene Bedeutung hat,
b) das Reaktionsprodukt aus Schritt a) einer Auftrennung unter Erhalt einer an 2-substituierten 4-Hydroxy-4-methyl-tetrahydropyranen der allgemeinen Formel (I) angereicherten Fraktion und einer Fraktion, die wenigstens eine der Verbindungen (V.1), (V.2) oder (V.3) und wenigstens eine Dioxanverbindung (VI) enthält, unterzieht,
c) die Fraktion, die wenigstens eine der Verbindungen (V.1), (V.2) oder (V.3) und wenigstens eine Dioxanverbindung (VI) enthält, einer Hydrierung unterzieht,
d) aus dem in Schritt c) erhaltenen Hydrierprodukt eine an 2-substituierten 4-Methyl-tetrahydropyranen (II) angereicherte Fraktion und eine an der wenigstens einen Dioxanverbindung (VI) angereicherte Fraktion isoliert.

Der vorliegenden Erfindung liegt die Aufgabe zugrunde, ein verbessertes Verfahren zur Herstellung 2-substituierter 4-Hydroxy-4-methyl-tetrahydropyrane zur Verfügung zu stellen, das eine effektive Herstellung in technischem Maßstab unter möglichst geringer Bildung unerwünschter und zu entsorgender Nebenprodukte ermöglicht.

Überraschenderweise wurde jetzt gefunden, dass diese Aufgabe durch eine Fahrweise unter Einsatz von wenigstens einem in Schlaufenfahrweise betriebenen Reaktor mit nachgeschalteter Trennkolonne gelöst wird. Dabei handelt es sich speziell um ein kontinuierliches Verfahren.

### ZUSAMMENFASSUNG DER ERFINDUNG

Gegenstand der Erfindung ist ein Verfahren zur Herstellung von 2-substituierten 4-Hydroxy-4-methyl-tetrahydropyranen der allgemeinen Formel (I) 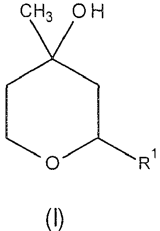 worin
- R¹: für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
umfassend eine Umsetzung von 3-Methylbut-3-en-1-ol der Formel (III) 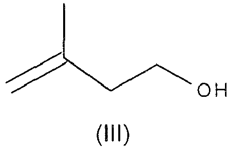 mit einem Aldehyd der Formel (IV)

R1-CHO (IV)

wobei R1 in der Formel (IV) die zuvor angegebene Bedeutung hat,
in Gegenwart eines sauren Katalysators, dadurch gekennzeichnet, dass die Umsetzung in einem Reaktor mit wenigstens einer nachgeschalteten Trennkolonne erfolgt, wobei ein Teilstrom des Austrags aus dem ersten Reaktor abgezogen und über einen externen Kreislauf in den Reaktor zurückgeführt wird, und wobei dem Teilstrom des Austrags aus dem Reaktor vor der Rückführung in den Reaktor Wärme entzogen wird.

### BESCHREIBUNG DER ERFINDUNG

Das erfindungsgemäße Verfahren weist die folgenden Vorteile auf:
- Das erfindungsgemäße Verfahren ermöglicht eine geringere thermische Belastung des Reaktorinhalts durch eine geringere Maximaltemperatur und/oder die Vermeidung von Temperaturspitzen.
- Das Verfahren ermöglicht somit höhere Ausbeuten und/oder eine höhere Selektivität bezüglich der Zielverbindungen.
- Eine geringere Maximaltemperatur und/oder die Vermeidung von Temperaturspitzen sind auch sicherheitstechnisch vorteilhaft und/oder ermöglichen eine längere Katalysatorstandzeit.
- Speziell der Einsatz eines Katalysatorfestbetts kann sich zusätzlich vorteilhaft auf die Katalysatorstandzeit auswirken. Somit werden langwierige An- und Abfahrvorgänge zum Tausch von verbrauchtem Katalysator bzw. zur Katalysatorregenerierung vermieden. Zudem verringert der Einsatz eines Katalysatorfestbetts auch die mechanische Belastung und Zerstörung des Katalysators.

Sofern im Folgenden nicht genauer angegeben, bezeichnen die Begriffe "2-substituiertes 4-Hydroxy-4-methyl-tetrahydropyran" und "2-(2-Methylpropyl)-4-hydroxy-4-methyl-tetrahydropyran" im Rahmen der Erfindung cis/trans-Gemische jedweder Zusammensetzung sowie die reinen Konformations-Isomere. Die zuvor genannten Begriffe bezeichnen weiterhin alle Enantiomere in Reinform sowie racemische und optisch aktive Gemische der Enantiomeren dieser Verbindungen.

Im Rahmen der vorliegenden Erfindung steht der Ausdruck geradkettiges oder verzweigtes Alkyl vorzugsweise für C₁-C₆-Alkyl und besonders bevorzugt für C₁-C₄-Alkyl. Alkyl steht insbesondere für Methyl, Ethyl, n-Propyl, Isopropyl, n-Butyl, Isobutyl (2-Methylpropyl), sec.-Butyl (1-Methylpropyl), tert.-Butyl (1,1-Dimethylethyl), n-Pentyl oder n-Hexyl. Speziell steht Alkyl für Methyl, Ethyl, n-Propyl, Isopropyl, oder Isobutyl.

Im Rahmen der vorliegenden Erfindung steht der Ausdruck geradkettiges oder verzweigtes Alkoxy vorzugsweise für C₁-C₆-Alkoxy und besonders bevorzugt für C₁-C₄-Alkoxy. Alkoxy steht insbesondere für Methoxy, Ethoxy, n-Propyloxy, Isopropyloxy, n-Butyloxy, Isobutyloxy, sec.-Butyloxy, tert.-Butyloxy, n-Pentyloxy oder n-Hexyloxy. Speziell steht Alkoxy für Methoxy, Ethoxy, n-Propyloxy, Isopropyloxy, oder Isobutyloxy.

Im Rahmen der vorliegenden Erfindung steht der Ausdruck geradkettiges oder verzweigtes Alkenyl vorzugsweise für C₂-C₆-Alkenyl und besonders bevorzugt für C₂-C₄-Alkenyl. Der Alkenylrest weist neben Einfachbindungen noch eine oder mehrere, bevorzugt 1 bis 3, besonders bevorzugt 1 oder 2 und ganz besonders bevorzugt eine ethylenische Doppelbindung auf. Alkenyl steht insbesondere für Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl oder 2-Methyl-2-propenyl.

Im Rahmen der Erfindung bezeichnet Cycloalkyl einen cycloaliphatischen Rest mit vorzugsweise 3 bis 10, besonders bevorzugt 5 bis 8, Kohlenstoffatomen. Beispiele für Cycloalkylgruppen sind insbesondere Cyclopropyl, Cyclobutyl, Cyclopentyl, Cyclohexyl, Cycloheptyl oder Cyclooctyl. Speziell steht Cycloalkyl für Cyclohexyl.

Substituierte Cycloalkylgruppen können in Abhängigkeit von der Ringgröße einen oder mehrere (z. B. 1, 2, 3, 4 oder 5) Substituenten aufweisen. Diese sind vorzugsweise unabhängig voneinander ausgewählt unter C₁-C₆-Alkyl und C₁-C₆-Alkoxy. Die Cycloalkylgruppen tragen im Falle einer Substitution vorzugsweise eine oder mehrere, beispielsweise eine, zwei, drei, vier oder fünf C₁-C₆-Alkylgruppen. Beispiele für substituierte Cycloalkylgruppen sind insbesondere 2- und 3-Methylcyclopentyl, 2- und 3-Ethylcyclopentyl, 2-, 3- und 4-Methylcyclohexyl, 2-, 3- und 4-Ethylcyclohexyl, 2-, 3- und 4-Propylcyclohexyl, 2-, 3- und 4-lsopropylcyclohexyl, 2-, 3- und 4-Butylcyclohexyl und 2-, 3- und 4-Isobutylcyclohexyl.

Der Ausdruck "Aryl" umfasst im Rahmen der vorliegenden Erfindung ein- oder mehrkernige aromatische Kohlenwasserstoffreste mit üblicherweise 6 bis 18, vorzugsweise 6 bis 14, besonders bevorzugt 6 bis 10 Kohlenstoffatomen. Beispiele für Aryl sind insbesondere Phenyl, Naphthyl, Indenyl, Fluorenyl, Anthracenyl, Phenanthrenyl, Naphthacenyl, Chrysenyl, Pyrenyl, etc., und speziell Phenyl oder Naphthyl.

Substituierte Aryle können in Abhängigkeit von der Anzahl und Größe ihrer Ringsysteme einen oder mehrere (z. B. 1, 2, 3, 4 oder 5) Substituenten aufweisen. Diese sind vorzugsweise unabhängig voneinander ausgewählt unter C₁-C₆-Alkyl und C₁-C₆-Alkoxy. Beispiele für substituierte Arylreste sind 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl und 2,4,6-Tri-tert.-butylphenyl.

Einer der Ausgangsstoffe für das erfindungsgemäße Verfahren ist 3-Methylbut-3-en-1-ol (Isoprenol) der Formel (III),

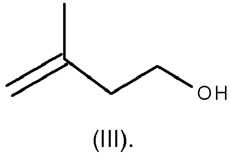

Isoprenol ist nach bekannten Verfahren aus Isobuten und Formaldehyd in jedem Maßstab gut zugänglich und kommerziell verfügbar. An die Reinheit, Qualität oder Herstellverfahren des erfindungsgemäß einzusetzenden Isoprenols sind keine besonderen Anforderungen zu stellen. Es kann in handelsüblicher Qualität und Reinheit in das erfindungsgemäße Verfahren eingesetzt werden. Bevorzugt setzt man Isoprenol ein, das eine Reinheit von 90 Gew.-% oder darüber hat, besonders bevorzugt solches mit einer Reinheit von 95 bis 100 Gew.-% und ganz besonders bevorzugt solches mit einer Reinheit von 97 bis 99,9 Gew.-% oder noch mehr bevorzugt 98 bis 99,8 Gew.-%.

Ein weiterer Ausgangsstoff für das erfindungsgemäße Verfahren ist ein Aldehyd der Formel (IV) R¹-CHO, wobei R¹ in der Formel (IV) die zuvor angegebene Bedeutung hat.

Bevorzugt steht R¹ in den Verbindungen der Formeln (I), (II) und (IV) für geradkettiges oder verzweigtes C₁-C₁₂-Alkyl oder geradkettiges oder verzweigtes C₂-C₁₂-Alkenyl. Besonders bevorzugt steht R¹ für geradkettiges oder verzweigtes C₁-C₆-Alkyl oder geradkettiges oder verzweigtes C₂-C₆-Alkenyl. In einer weiteren bevorzugten Ausführung steht R¹ für Phenyl.

Erfindungsgemäß bevorzugte Bedeutungen für den Rest R¹ sind somit beispielsweise Methyl, Ethyl, n-Propyl, Isopropyl, n-Butyl, Isobutyl, n-Pentyl, n-Hexyl oder n-Heptyl, bevorzugt Methyl, Ethyl, n-Propyl, Isopropyl, n-Butyl, Isobutyl, ganz besonders bevorzugt Isobutyl (2-Methylpropyl).

Besonders bevorzugt steht der Rest R¹ für Isobutyl oder Phenyl.

Bevorzugt einzusetzende Aldehyde der Formel (IV) sind: Acetaldehyd, Valeraldehyd, Isovaleraldehyd, Pentanal, Hexanal, Heptanal, Benzaldehyd, Citral, Citronellal. Erfindungsgemäß ganz besonders bevorzugt einzusetzende Aldehyde der Formel (IV) sind Isovaleraldehyd und Benzaldehyd, insbesondere Isovaleraldehyd.

Die Umsetzung der Verbindungen (III) und (IV) erfolgt in einer Anordnung aus einem Reaktor mit wenigstens einer nachgeschalteten Trennkolonne, d. h. der Reaktor und die Trennkolonne sind in Reihe geschaltet. In einer erfindungsgemäßen Anordnung kann der einzelne Reaktor auch durch zwei oder mehr parallel geschaltete Reaktoren ersetzt werden. In einer weiteren Ausgestaltung können auch mehrere Trennkolonnen parallel oder in Reihe geschaltet sein.

Ein Teilstrom des Austrags aus dem Reaktor wird abgezogen und über einen externen Kreislauf in den Reaktor zurückgeführt. Diese Betriebsweise wird hier und im Folgenden auch als Schlaufenfahrweise bezeichnet. Die Schlaufenfahrweise kann zusätzlich rückvermischende Einbauten und/oder Rühreinrichtungen im Reaktor umfassen.

Vorzugsweise erfolgt die Umsetzung kontinuierlich. Das bedeutet, dass alle in Reihe geschalteten Komponenten, d. h. Reaktor und Trennkolonne(n), jeweils kontinuierlich betrieben werden.

In einer geeigneten Ausführungsform erfolgt die Umsetzung in Gegenwart eines Lösungsmittels. Gegebenenfalls werden zur Durchführung der erfindungsgemäßen Umsetzung die Verbindungen der Formeln (III) und (IV), hier und im Folgenden auch als Ausgangsstoffe oder Edukte bezeichnet, jeweils in Form eines Gemisches mit einem geeigneten Lösungsmittel zugeführt. Bevorzugt werden beide Ausgangsstoffe (III) und (IV) im gleichen Lösungsmittel vorgelegt. Bei dem Lösungsmittel handelt es sich vorzugsweise um Wasser oder ein unter den Reaktionsbedingungen inertes Lösungsmittel wie beispielsweise tert-Butylmethylether, Cyclohexan, Toluol, Hexan oder Xylol. Die genannten Lösungsmittel können alleine oder in Form von Gemischen eingesetzt werden. In einer bevorzugten Ausgestaltung führt man die Umsetzung ohne Zusatz eines organischen Lösungsmittels durch. In einer besonders bevorzugten Ausgestaltung erfolgt die Umsetzung in Gegenwart von Wasser.

In der erfindungsgemäßen Ausgestaltung wird dem Teilstrom des Austrags aus dem Reaktor vor der Rückführung in den Reaktor Wärme entzogen.

In einer geeigneten Ausgestaltung wird dem Austrag aus dem Reaktor vor der Zuführung in die Trennkolonne zusätzlich Wärme zugeführt. Die Stromteilung für die Rückführung in den Reaktor wird in diesem Fall vor einer Zwischenkühlung erfolgen.

Gemäß einer weiteren Ausführungsform wird dem Austrag aus dem Kopf der wenigstens einen Trennkolonne zumindest ein Teilstrom entnommen und über eine externe Rückführung in den Reaktor zurückgeführt. Der Kopfstrom aus der destillativen Trennung, der im Wesentlichen aus den nicht umgesetzten Edukten Aldehyd, Alkohol und Wasser besteht, kann vorteilhafter Weise gemeinsam mit den reinen Eduktströmen oder getrennt davon in den Reaktor zurückgeführt werden.

In einer bevorzugten Ausführungsform des erfindungsgemäßen Verfahrens wird die Umsetzung im Reaktor adiabatisch durchgeführt wird.

Der Begriff "adiabatisch" wird im Rahmen der vorliegenden Erfindung im technischen und nicht im physikalisch-chemischen Sinne verstanden. So erfährt das Reaktionsgemisch beim Strömen durch den Reaktor auf Grund der exothermen Reaktion in der Regel eine Temperaturerhöhung. Unter adiabatischer Reaktionsführung wird eine Vorgehensweise verstanden, bei der die bei der Reaktion freiwerdende Wärmemenge von der Reaktionsmischung im Reaktor aufgenommen und keine Kühlung durch Kühlvorrichtungen erfolgt. Somit wird die Reaktionswärme weitestgehend mit dem Reaktionsgemisch aus dem Reaktor abgeführt. Es liegt auf der Hand, dass ein Restanteil durch natürliche Wärmeleitung bzw. -strahlung vom Reaktor an die Umgebung abgegeben wird.

In einer alternativen Ausführungsform des erfindungsgemäßen Verfahrens wird der Reaktor weitgehend isotherm betrieben.

Unter "weitgehend isotherm betrieben" soll im Rahmen der vorliegenden Erfindung verstanden werden, dass in der jeweiligen Reaktionszone ein enges Temperaturintervall eingehalten wird. Wird der Reaktor "weitgehend isotherm betrieben", so soll im Rahmen der vorliegenden Erfindung darunter verstanden werden, dass das Temperaturintervall Δ T im Reaktor kleiner als die adiabatische Temperaturerhöhung ist. Für das Temperaturintervall im Reaktor gilt bevorzugt Δ T ≤ 12 K, besonders bevorzugt Δ T ≤ 10 K.

Für eine weitgehend isotherme Betriebsweise des Reaktors sind geeigneter Weise Wärmeübertragungsflächen im Inneren des verwendeten Reaktors angeordnet, d. h. der verwendete Reaktor umfasst einen intern angeordneten Wärmeübertrager.

Gemäß einer besonders bevorzugten Ausführungsform ist der verwendete Reaktor ein Festbettreaktor.

Vorzugsweise erfolgt die Umsetzung in Gegenwart eines sauren Katalysators, der ausgewählt ist unter Salzsäure, Schwefelsäure, Methansulfonsäure, p-Toluolsulfonsäure und stark sauren Kationenaustauschern. Insbesondere wird die Umsetzung in Gegenwart eines stark sauren Kationenaustauschers durchgeführt.

Bevorzugt werden der Alkohol der Formel (III) und der Aldehyd der Formel (IV) in einem molaren Verhältnis im Bereich von 0,7 : 1 bis 2 : 1 eingesetzt.

Vorzugsweise werden der Alkohol der Formel (III) und der Aldehyd der Formel (IV) in Gegenwart von mindestens 3 Gew.-%, besonders bevorzugt von mindestens 5 Gew.-% Wasser umgesetzt. Der Alkohol der Formel (III) und der Aldehyd der Formel (IV) werden beispielsweise in Gegenwart von 3 Gew.-% bis 15 Gew.-% Wasser, bevorzugt von 5 Gew.-% bis 12 Gew.-% umgesetzt. Die angegebenen vorstehenden Gew.-% sind dabei bezogen auf die Menge des Reaktionsgemisches, bestehend aus den Komponenten der Formeln (III) und (IV) sowie Wasser.

In der Regel führt man die Umsetzung des Alkohols der Formel (III) mit dem Aldehyd der Formel (IV) in Gegenwart von etwa mindestens 10 Mol-% Wasser durch, wobei sich die Menge an Wasser auf die Menge des gegebenenfalls im Unterschuss eingesetzten Ausgangsstoffes, oder im Fall einer äquimolaren Umsetzung auf die Stoffmenge eines der beiden Ausgangsstoffe bezieht. Oberhalb des angegebenen Wertes kann die Menge an Wasser frei gewählt werden und ist nur durch verfahrenstechnische oder ökonomische Aspekte limitiert. Wasser kann auch in großem, beispielsweise in 10- bis 100-fachem Überschuss oder sogar darüber eingesetzt werden. Vorzugsweise bereitet man ein Gemisch aus dem Alkohol der Formel (III) und dem Aldehyd der Formel (IV) mit der gewählten Menge Wasser, so dass das zugegebene Wasser in dem Gemisch gelöst bleibt, d. h. dass kein zweiphasiges System vorliegt.

In einer geeigneten Ausgestaltung werden die Ausgangsstoffe in Gegenwart von mindestens 25 Mol-%, bevorzugt von mindestens 50 Mol-% Wasser umgesetzt. Beispielsweise werden die Ausgangsstoffe in Gegenwart von 25 bis 150 Mol-%, bevorzugt von 40 bis 150 Mol-%, besonders bevorzugt von 50 bis 140 Mol-%, insbesondere von 50 bis 80 Mol-% Wasser umgesetzt. Dabei bezieht sich die Menge an eingesetztem Wasser auf die Stoffmenge des gegebenenfalls im Unterschuss eingesetzten Ausgangsstoffes oder im Fall einer äquimolaren Umsetzung auf die Stoffmenge eines der beiden.

In einer geeigneten Ausführungsform des erfindungsgemäßen Verfahrens wird die Umsetzung bei einer Temperatur im Bereich von 0 °C bis 70 °C, bevorzugt im Bereich von 20 °C bis 70 °C, besonders bevorzugt im Bereich von 20 °C bis 60 °C durchgeführt.

In einer ebenfalls geeigneten Ausführungsform des erfindungsgemäßen Verfahrens wird die Umsetzung bei einem Druck im Bereich von 1 bar bis 15 bar durchgeführt.

In einer geeigneten Ausführungsform kann in dem eingesetzten Reaktor eine zusätzliche Durchmischung erfolgen. Eine zusätzliche Durchmischung ist insbesondere vorteilhaft, wenn die Reaktion bei großen Verweilzeiten des Reaktionsgemischs erfolgt. Geeignet sind sowohl statische als auch dynamische Mischvorrichtungen. Geeignete Mischvorrichtungen sind dem Fachmann hinlänglich bekannt. Zur Durchmischung können bevorzugt die Feedströme über geeignete Mischvorrichtungen, wie Düsen, in den Reaktor eingespeist werden.

Die zuvor beschriebene Schlaufenfahrweise eignet sich besonders vorteilhaft zur Regulierung der Reaktionstemperatur und des Wärmeübergangs zwischen Reaktionsmedium, Apparatewänden und Umgebung. Eine weitere Möglichkeit zur Steuerung der Wärmebilanz besteht in der Regelung der Eintrittstemperatur der Edukte bzw. des Rückführstromes. So führt eine tiefere Temperatur des eintretenden Feeds in der Regel zu einer verbesserten Abführung der Reaktionswärme. Beim Nachlassen der Katalysatoraktivität kann die Eintrittstemperatur höher gewählt werden, um eine höhere Reaktionsgeschwindigkeit zu erreichen und somit die nachlassende Katalysatoraktivität zu kompensieren. Die Standzeit des eingesetzten Katalysators kann so in vorteilhafter Weise erhöht werden.

Der Rückführstrom wird im Allgemeinen chemisch unverändert in das Reaktionssystem zurückgeführt. Sofern gewünscht, können die Temperatur und/oder der Druck vor der Zurückführung auf die gewünschten Werte eingestellt werden. Die Einspeisung des Rückführstroms in den Reaktor kann gemeinsam mit einem, zwei oder drei der Feedströme oder separat davon erfolgen. Das Gewichtsmengenverhältnis des in den Reaktor eingespeisten Rückführstroms zum Gesamt-Feedstrom liegt vorzugsweise in einem Bereich von 1 : 1 bis 50 : 1, besonders bevorzugt in einem Bereich von 2 : 1 bis 30 : 1, insbesondere im Bereich von 5 : 1 bis 20 : 1.

Das Kopfprodukt, das im Wesentlichen aus den Edukten Isovaleraldehyd und Isoprenol sowie Wasser besteht, wird am Kopf der Trennkolonne abgezogen und kann vorteilhafterweise in den Reaktor zurückgeführt werden. Das Kopfprodukt wird im Allgemeinen chemisch unverändert in das Reaktionssystem zurückgeführt. Sofern gewünscht, können die Temperatur und/oder der Druck vor der Zurückführung auf die gewünschten Werte eingestellt werden. Die Zurückführung des Kopfproduktes in den Reaktor kann gemeinsam mit einem, zwei oder drei der Feedströme oder separat davon erfolgen.

Durch die beschriebene Rückführung von Ausgangsstoffen kann der Feedstrom in den Reaktor um die entsprechende Menge Edukte reduziert werden.

Bevorzugt erfolgt die Umsetzung in Gegenwart eines stark sauren Kationenaustauschers. Unter dem Begriff stark saurer Kationenaustauscher wird dabei ein Kationenaustauscher in der H⁺-Form verstanden, der stark saure Gruppen aufweist. Bei den stark sauren Gruppen handelt es sich in der Regel um Sulfonsäuregruppen. Die sauren Gruppen sind in der Regel angebunden an eine Polymermatrix, die z. B. gelförmig bzw. makroporös sein kann. Eine bevorzugte Ausführungsform des erfindungsgemäßen Verfahrens ist dementsprechend dadurch gekennzeichnet, dass man einen stark sauren, Sulfonsäuregruppen aufweisenden Kationenaustauscher einsetzt. Geeignete stark saure Kationenaustauscher sind in der WO 2010/133473 und WO 2011/154330 beschrieben, worauf hier in vollem Umfang Bezug genommen wird.

Geeignet für den Einsatz sind stark saure Ionenaustauscher (wie z. B. Amberlyst ™, Amberlite ®, Dowex ®, Lewatit ®, Purolite ®, Serdolit ®), die auf Polystyrol basieren und die Copolymere aus Styrol und Divinylbenzol als Trägermatrix mit Sulfonsäuregruppen in H⁺-Form enthalten sowie mit Sulfonsäuregruppen (-SO₃H) funktionalisierte Ionenaustauschergruppen. Die Ionenaustauscher unterscheiden sich im Aufbau ihrer Polymergerüste, und man unterscheidet gelförmige und makroporöse Harze. In einer speziellen Ausführung wird ein perfluoriertes polymeres Ionenaustauscherharz eingesetzt. Derartige Harze werden z. B. unter der Bezeichnung Nafion® von der Firma Du-Pont vertrieben. Als Beispiel für ein solches perfluoriertes polymeres Ionenaustauscherharz sei Nafion® NR-50 genannt.

Für die Umsetzung geeignete kommerziell verfügbare stark saure Kationenaustauscher sind beispielsweise unter den Handelsnamen Lewatit ® (Lanxess), Purolite ® (The Purolite Company), Dowex ® (Dow Chemical Company), Amberlite ® (Rohm and Haas Company), Amberlyst ™ (Rohm and Haas Company) bekannt. Bevorzugte stark saure Kationenaustauscher sind: Lewatit ® K 1221, Lewatit ® K 1461, Lewatit ® K 2431, Lewatit ® K 2620, Lewatit ® K 2621, Lewatit ® K 2629, Lewatit ® K 2649, Amberlite ® FPC 22, Amberlite ® FPC 23, Amberlite ® IR 120, Amberlyst ™ 131, Amberlyst ™ 15, Amberlyst ™ 31, Amberlyst ™ 35, Amberlyst ™ 36, Amberlyst ™ 39, Amberlyst ™ 46, Amberlyst ™ 70, Purolite ® SGC650, Purolite ® C100H, Purolite ® C150H, Dowex ® 50X8, Serdolit ® rot und Nation ® NR-50.

Die stark sauren lonentauscherharze werden in der Regel mit Salzsäure und/oder Schwefelsäure regeneriert.

In einer speziellen Ausführung werden das 3-Methylbut-3-en-ol (III) und der Aldehyd (IV) in Gegenwart eines stark sauren Kationenaustauschers und in Gegenwart von Wasser umgesetzt. Prinzipiell kann das Reaktionsgemisch bereits geringe Mengen Wasser enthalten, das durch die Dehydratisierung des Verfahrensproduktes der Formel (I) als mögliche Nebenreaktion freigesetzt werden kann. Nach einer speziellen Ausführung wird dem Reaktionsgemisch neben Isoprenol (III) und dem Aldehyd der Formel (IV) sowie etwaigem Wasser aus der Reaktion zusätzlich noch Wasser zugesetzt.

Vorzugsweise werden der Alkohol der Formel (III) und der Aldehyd der Formel (IV) in Gegenwart von mindestens 3 Gew.-%, besonders bevorzugt von mindestens 5 Gew.-% Wasser umgesetzt. Der Alkohol der Formel (III) und der Aldehyd der Formel (IV) werden beispielsweise in Gegenwart von 3 Gew.-% bis 15 Gew.-% Wasser, bevorzugt von 5 Gew.-% bis 12 Gew.-% umgesetzt. Die angegebenen vorstehenden Gew.-% sind dabei bezogen auf die Gesamtmenge des Reaktionsgemisches, bestehend aus den Komponenten der Formeln (III) und (IV) sowie Wasser.

Oberhalb des angegebenen Wertes kann die Menge an Wasser frei gewählt werden und ist, wenn überhaupt, nur durch verfahrenstechnische oder ökonomische Aspekte begrenzt und kann durchaus in großem, beispielsweise in 5- bis 15-fachem Überschuss oder auch darüber eingesetzt werden. Vorzugsweise bereitet man ein Gemisch aus Isoprenol (III) und dem Aldehyd der Formel (IV), vorzugsweise Isovaleraldehyd, mit der zuzusetzenden Menge Wasser, so dass das zugegebene Wasser in dem Gemisch aus Isoprenol und dem Aldehyd gelöst bleibt d. h. kein zweiphasiges System vorliegt.

Üblicherweise setzt man im Rahmen dieser Ausführungsform des erfindungsgemäßen Verfahrens die Ausgangsstoffe Isoprenol (III) und den Aldehyd der Formel (IV) in Gegenwart von mindestens 25 Mol-%, bevorzugt von mindestens 50 Mol-% um. Beispielsweise werden die Ausgangsstoffe in Gegenwart von 25 bis 150 Mol-%, bevorzugt von 40 bis 150 Mol-%, besonders bevorzugt von 50 bis 140 Mol-%, insbesondere von 50 bis 80 Mol-% Wasser umgesetzt. Dabei bezieht sich die Menge an eingesetztem Wasser auf die Stoffmenge des gegebenenfalls im Unterschuss eingesetzten Ausgangsstoffes oder im Fall einer äquimolaren Umsetzung auf die Stoffmenge eines der beiden.

Zur Umsetzung von Isoprenol (III) mit dem Aldehyd (IV) kann man die genannten Ausgangsstoffe und gegebenenfalls das zugesetzte Wasser mit dem sauren Kationenaustauscher in Kontakt bringen. Vorzugsweise werden Isoprenol (III), Aldehyd (IV) und gegebenenfalls das zugesetzte Wasser in Form eines Gemisches eingesetzt. Die genannten Ausgangsstoffe, d. h. Isoprenol (III) und der Aldehyd (IV) und das in der vorstehenden Menge einzusetzende Wasser können in beliebiger Reihenfolge miteinander in Kontakt gebracht bzw. gemischt werden.

Die Menge an stark saurem Kationenaustauscher ist nicht kritisch und kann unter Berücksichtigung des wirtschaftlichen und verfahrenstechnischen Aspektes in breiten Grenzen frei gewählt werden. Die Umsetzung kann dementsprechend sowohl in Gegenwart katalytischer Mengen als auch in Gegenwart großer Überschüsse des stark sauren Kationenaustauschers durchgeführt werden. Die genannten stark sauren Kationenaustauscher können sowohl einzeln oder auch in Form von Gemischen eingesetzt werden.

Die Katalysatorbelastung liegt beispielsweise im Bereich von 50 bis 2500 mol pro m³ Katalysator und h, bevorzugt im Bereich von 100 bis 2000 mol pro m³ Katalysator und h, insbesondere im Bereich von 130 bis 1700 mol pro m³ Katalysator und h, wobei sich die Stoffmenge in mol auf den Ausgangsstoff der Formel (IV) bezieht.

Die Umsetzung in Gegenwart eines stark sauren Kationenaustauschers kann wahlweise auch zusätzlich in Gegenwart eines unter den Reaktionsbedingungen inerten Lösungsmittels durchgeführt werden. Geeignete Lösungsmittel sind beispielsweise tert-Butylmethylether, Cyclohexan, Dekalin, Hexan, Heptan, Ligroin, Petrolether, Toluol oder Xylol. Die genannten Lösungsmittel können alleine oder in Form von Gemischen untereinander eingesetzt werden. Bevorzugt führt man die Umsetzung in Gegenwart eines stark sauren Kationenaustauschers ohne Zusatz eines organischen Lösungsmittels durch.

Bevorzugt wird die Umsetzung von Isoprenol (III) mit dem gewählten Aldehyd (IV) in Gegenwart von Wasser und in Gegenwart eines stark sauren Kationentauschers bei einer Temperatur im Bereich von 0 bis 70 °C, besonders bevorzugt bei einer Temperatur im Bereich von 20 bis 70 °C und insbesondere bei einer Temperatur im Bereich von 20 bis 60 °C durchgeführt. Dabei handelt es sich um die Temperatur des Reaktionsgemisches.

Das aus dem Reaktor abgezogene Reaktionsgemisch wird einer destillativen Auftrennung in einer dem Reaktor nachgeschalteten Trennkolonne unterzogen. Geeignete Trennkolonnen umfassen Destillationskolonnen, wie Bodenkolonnen, die mit Glocken, Siebplatten, Siebböden, Packungen, Füllkörpern, Ventilen, Seitenabzügen, etc. ausgerüstet sein können, Verdampfer, wie Dünnschichtverdampfer, Fallfilmverdampfer, Zwangsumlaufverdampfer, Sambay-Verdampfer, etc. und Kombinationen davon.

Die Trennkolonne kann trennwirksame Einbauten aufweisen, die vorzugsweise ausgewählt sind unter Trennböden, geordneten Packungen, z. B. Blech- oder Gewebepackungen, wie Sulzer Mellapak®, Sulzer BX, Kühni Rombopak, Montz B1 oder Montz A3, oder regellosen Schüttungen von Füllkörpern, wie z. B. Dixon-Ringen, Raschig-Ringen, High-Flow-Ringen oder Raschig-Super-Ringen. Besonders bewährt haben sich geordnete Packungen, vorzugsweise Blech- oder Gewebepackungen, mit einer spezifischen Oberfläche von 100 bis 750 m²/m³, insbesondere 250 bis 500 m²/m³. Sie gestalten hohe Trennleistungen bei niedrigen Druckverlusten.

Vorzugsweise wird zur Auftrennung eine Trennkolonne eingesetzt, die
- eine Zulaufsäule mit oberhalb der Zulaufstelle gelegenem Verstärkungsteil und unterhalb der Zulaufstelle gelegenem Abtriebsteil,
- eine mit dem oberen Ende des Verstärkungsteils kommunizierende obere Vereinigungssäule und eine mit dem unteren Ende des Abtriebsteils kommunizierende untere Vereinigungssäule, und
- eine mit der oberen Vereinigungssäule und der unteren Vereinigungssäule kommunizierende Abzugssäule
umfasst.

Vorzugsweise erfolgt die Auftrennung in der Trennkolonne, indem man
i. das Reaktionsprodukt aus dem Reaktor in eine Zulaufsäule mit oberhalb der Zulaufstelle gelegenem Verstärkungsteil und unterhalb der Zulaufstelle gelegenem Abtriebsteil einführt,
ii. eine mit dem oberen Ende des Verstärkungsteils kommunizierende obere Vereinigungssäule mit Kondensator am oberen Säulenende und eine mit dem unteren Ende des Abtriebsteils kommunizierende untere Vereinigungssäule mit Heizvorrichtung am unteren Säulenende vorsieht,
iii. eine mit der oberen Vereinigungssäule und der unteren Vereinigungssäule kommunizierende Abzugssäule vorsieht, die wenigstens einen Seitenabzug aufweist,
iv. aus der Abzugssäule am Kopf oder im oberen Bereich die Verbindungen abzieht, die leichter als die-2-substituierten 4-Hydroxy-4-methyl-tetrahydropyrane (I) sieden,
v. als wenigstens einen Seitenabzug zumindest einen Teil der 2-substituierten 4-Hydroxy-4-methyl-tetrahydropyrane (I) abzieht und
vi. im Sumpf oder im unteren Bereich der unteren Vereinigungssäule die 2-substituierten 4-Hydroxy-4-methyl-tetrahydropyrane (I), die nicht als Seitenabzug abgezogen werden, sowie die Verbindungen, die höher als die 2-substituierten 4-Hydroxy-4-methyl-tetrahydropyrane (I) sieden, abzieht.

In einer bevorzugten Ausführungsform enthält der aus der Abzugssäule am Kopf oder im oberen Bereich entnommene Abzug im Wesentlichen:
- nicht umgesetztes 3-Methylbut-3-en-1-ol der Formel (III),
- nicht umgesetzten Aldehyd der Formel (IV),
- geringe Mengen oder keine 4-Hydroxy-4-methyl-tetrahydropyrane der Formel (I),
- Wasser.

Das so erhaltene Kopfprodukt kann gegebenenfalls einer Phasentrennung zur Abtrennung der Hauptmenge des Wassers unterzogen werden. Abgesehen von einer solchen Phasentrennung kann das so erhaltene Kopfprodukt in der Regel ohne weitere Aufarbeitung in den Reaktor zurückgeführt werden. Sofern gewünscht kann das Kopfprodukt einer weiteren Aufarbeitung zur Abtrennung wenigstens eines Teils der von den Edukten (III) und (VI) verschiedenen Komponenten unterzogen werden. Dazu kann das Kopfprodukt z. B. einer weiteren destillativen Auftrennung unterzogen werden.

In einer bevorzugten Ausführungsform wird aus der Abzugssäule ein Seitenstrom oder werden aus der Abzugssäule zwei Seitenströme abgezogen. In einer speziellen Ausführung wird aus der Abzugssäule nur ein Seitenstrom abgezogen.

Werden der Trennkolonne mehrere Abzüge entnommen, die 2-substituierte 4-Hydroxy-4-methyl-tetrahydropyrane (I) enthalten, z. B. zwei verschiedene Seitenabzüge oder ein Seitenabzug und ein Sumpfabzug, so unterscheiden sich diese in der Regel hinsichtlich der Zusammensetzung der Stereoisomeren. Somit gelingt die Isolierung einer gegenüber dem aus dem Reaktor abgezogenen Reaktionsprodukt an cis-Diastereomeren angereicherten Fraktion und einer an trans-Diastereomeren angereicherten Fraktion. Bei ausreichender Trennleistung der eingesetzten Trennkolonne kann gegebenenfalls zumindest eines der Diastereomeren in reiner Form erhalten werden.

Die Zulaufsäule, Abzugssäule, obere Vereinigungssäule und untere Vereinigungssäule können diskrete Bauelemente sein oder als Abschnitt oder Kammer einer Destillationskolonne ausgebildet sein, die mehrere Funktionen vereint. Der Ausdruck "kommunizierende Kolonnen" bedeutet, dass zwischen ihnen ein Austausch sowohl von aufsteigendem Brüden als auch von ablaufendem Kondensat erfolgt.

In einer bevorzugten Ausführungsform des erfindungsgemäßen Verfahrens erfolgt die destillative Auftrennung in einer Anordnung von Trennkolonnen, die eine Trennwandkolonne oder eine Zusammenschaltung von mindestens zwei thermisch gekoppelten konventionellen Destillationskolonnen umfasst.

Trennwandkolonnen sind spezielle Destillationskolonnen mit mindestens einer Zulaufstelle und mindestens drei Entnahmestellen. Zwischen Verdampfer und Kondensator befindet sich der sogenannte Rektifizierbereich, in dem sich ein Teil des im Kondensator gebildeten Kondensats flüssig als Rücklauf im Gegenstrom zu den aus der Verdampfungseinrichtung aufsteigenden Dämpfen abwärts bewegt. Der Rektifizierbereich enthält in einem Teilbereich der Kolonne unterhalb und/oder oberhalb der Zulaufstelle mindestens eine in Längsrichtung wirkende Trenneinrichtung (Trennwand) zur Verhinderung einer Quervermischung von Flüssigkeits- und/oder Brüdenstrom (Dampfstrom), womit eine destillative Trennung von Stoffgemischen ermöglicht wird. Das Grundprinzip der Trennwandkolonnen ist seit langem bekannt und beispielsweise in der US 2,471,134, in der EP-A-0 122 367 oder in G. Kaibel, Chem. Eng. Technol. Vol. 10, 1987, Seite 92 bis 98 beschrieben.

Der allgemeine Aufbau einer Trennwandkolonne umfasst mindestens eine seitliche Zulaufstelle auf einer Seite der Trennwand und mindestens drei Entnahmestellen, von denen sich mindestens eine jenseits der Trennwand befindet. Da bei dieser Bauart im Bereich der Trennwand eine Quervermischung von Flüssigkeits- und/oder Brüdenstrom verhindert wird, ist es möglich, die Seitenprodukte in reiner Form zu erhalten. Hierdurch verringert sich bei der Auftrennung von Vielstoffgemischen im Allgemeinen die Zahl der insgesamt benötigten Destillationskolonnen. Zudem können beim Einsatz von Trennwandkolonnen gegenüber einer einfachen Hintereinanderschaltung zweier konventioneller Destillationskolonnen Investitionskosten sowie Energie eingespart werden (siehe M. Knott, Process Engineering, Vol. 2, 1993, February, Seite 33 bis 34).

Als konventionelle Destillationskolonnen werden im Sinne der Erfindung alle Destillationskolonnen bezeichnet, die keine Trennwand enthalten. Bei thermisch gekoppelten konventionellen Destillationskolonnen werden Massen- und Energieströme wechselseitig ausgetauscht. Somit ist gegenüber einer einfachen Hintereinanderschaltung konventioneller Destillationskolonnen eine deutliche Einsparung von Energie möglich. Bevorzugt als Alternative zur Trennwandkolonne ist eine Verschaltung zweier thermisch gekoppelter Destillationskolonnen. Eine Übersicht verschiedener Anordnungen ist beispielsweise in G. Kaibel et al., Chem.-Ing.-Tech., Vol. 61, 1989, Seite 16 bis 25 und G. Kaibel et al., Gas Separation & Purification, Vol. 4, 1990, June, Seiten 109 bis 114, gegeben.

In einer ersten bevorzugten Ausführungsform verwendet man zur Destillation eine Destillationskolonne mit einer thermisch gekoppelten Vorkolonne, d. h. die Abzugssäule, die obere Vereinigungssäule und die untere Vereinigungssäule sind als einstückige Destillationskolonne ausgebildet, und die Zulaufsäule ist als Vorkolonne zur Destillationskolonne ausgebildet. In einer zweiten bevorzugten Ausführungsform verwendet man eine Destillationskolonne mit einer thermisch gekoppelten Nachkolonne, d. h. die Zulaufsäule, die obere Vereinigungssäule und die untere Vereinigungssäule sind als einstückige Destillationskolonne ausgebildet und die Abzugssäule ist als Nachkolonne zu der Destillationskolonne ausgebildet. Destillationskolonnen mit beigeschalteten Hilfskolonnen sind bekannt und z. B. in Chem. Eng. Res. Des., Part A: Trans IChemE, März 1992, S. 118-132, "The design and optimisation of fully thermally coupled distillation columns", beschrieben.

Es hat sich als günstig erwiesen, aus dem aus dem Reaktor abgezogenen Reaktionsgemisch vor dem Einführen in die Zulaufsäule zumindest einen Teil der leichter als die 2-substituierten 4 Hydroxy-4-methyl-tetrahydropyrane (I) siedenden Verbindungen zu entfernen. In einer speziellen Ausführungsform wird daher zur destillativen Auftrennung des Reaktionsprodukts aus dem Reaktor eine Anordnung von Destillationskolonnen eingesetzt, die eine vorgeschaltete konventionelle Destillationskolonne und eine nachgeschaltete Trennwandkolonne oder eine nachgeschaltete Zusammenschaltung von zwei thermisch gekoppelten konventionellen Destillationskolonnen umfasst.

Bevorzugt wird zur destillativen Auftrennung
- das Reaktionsgemisch aus dem Reaktor zunächst einer Auftrennung in einer konventionellen Destillationskolonne unterzogen, wobei ein erstes Kopfprodukt, das an den Verbindungen der Formeln (III) und (IV) abgereichert ist und im Wesentlichen keine Verbindungen der Formel (I) enthält, und ein erstes Sumpfprodukt, das an den Verbindungen (III) und (IV) angereichert ist und die Hauptmenge der Verbindungen der Formel (I) enthält, erhalten wird, und
- das erste Sumpfprodukt einer Auftrennung in einer Trennwandkolonne oder in einer Zusammenschaltung von zwei thermisch gekoppelten konventionellen Destillationskolonnen unterzogen, wobei ein zweites Kopfprodukt, das die nicht im ersten Kopfprodukt enthaltenen Verbindungen (III) und (IV) sowie gegebenenfalls geringe Mengen der Verbindungen der Formel (I) enthält, ein Seitenstrom, der im Wesentlichen aus Verbindungen der Formel (I) besteht, und ein zweites Sumpfprodukt, das die Verbindungen der Formel (I) enthält, die nicht im Kopfprodukt und nicht im Seitenstrom enthalten sind, erhalten wird.

Das erste Kopfprodukt enthält im Wesentlichen keine Verbindungen der Formel (I). Das heißt, dass der Anteil der Verbindungen der Formel (I) im ersten Kopfprodukt höchstens 5 Gew.-%, besonders bevorzugt höchstens 2 Gew.-%, insbesondere höchstens 1 Gew.-%, speziell höchstens 0,1 Gew.-%, bezogen auf das Gesamtgewicht des ersten Kopfprodukts, beträgt. In einer speziellen Ausführung enthält das erste Kopfprodukt keine Verbindungen der Formel (I).

Das zweite Kopfprodukt kann beispielsweise 0,1 bis 25 Gew.-%, besonders bevorzugt 0,2 bis 20 Gew.-%, insbesondere 0,3 bis 15 Gew.-%, speziell 0,5 bis 10 Gew.-%, Verbindungen der Formel (I), bezogen auf das Gesamtgewicht des zweiten Kopfprodukts, enthalten.

In einer speziellen Ausführung besteht der Seitenstrom nur aus Verbindungen der Formel (I).

In einer speziellen Ausführung besteht das zweite Sumpfprodukt nur aus Verbindungen der Formel (I). Alternativ kann das zweite Sumpfprodukt Verbindungen enthalten, die höher sieden als die Verbindungen der Formel (I).

Vorzugsweise wird nach dieser Ausführungsform das erste Kopfprodukt (insbesondere die an Wasser abgereicherte organische Phase des ersten Kopfprodukts) und/oder das zweite Kopfprodukt zur Umsetzung in den Reaktor zurückgeführt. Dabei ist es unkritisch, wenn das zweite Kopfprodukt noch geringe Mengen der Verbindungen der Formel (I) enthält, da diese die Umsetzung im Reaktor in der Regel unverändert durchlaufen und anschließend gegebenenfalls abgetrennt und verwertet werden können.

In der Regel werden sich bei dieser Ausführungsform das Seitenprodukt und das zweite Sumpfprodukt bezüglich des Anteils der Stereoisomeren der Verbindungen der Formel (I) unterscheiden.

Die Aufarbeitung des Reaktionsaustrags zur Gewinnung des Wertprodukts kann nach üblichen, dem Fachmann bekannten Verfahren erfolgen. Bevorzugt umfasst die Aufarbeitung des Reaktionsgemisches wenigstens einen Destillationsschritt. Der Reaktionsaustrag kann in bekannter Weise durch Destillation bzw. Rektifikation aufgetrennt werden, um so das Wertprodukt zu erhalten. Beispielsweise kann die Aufarbeitung analog zu der in der WO 2011/154330, auf die hier in vollem Umfang Bezug genommen wird, beschriebenen Methode erfolgen.

### FIGURENBESCHREIBUNG

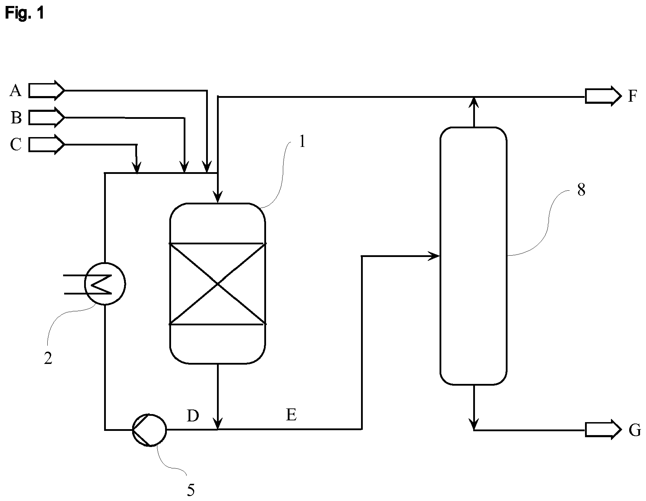

Das erfindungsgemäße Verfahren wird anhand der Figur 1 im Folgenden näher erläutert, ohne es auf diese Ausführungsform einzuschränken.

In der Figur 1 werden folgende Bezugszeichen verwendet:
- 1: Reaktor
- 2: Kühler
- 5: Pumpe
- 8: Trennkolonne

- A: Isoprenol
- B: Aldehyd
- C: Wasser D Rückführstrom
- E: Feed
- F: Kopfprodukt
- G: Edukt

Das erfindungsgemäße Verfahren kann mit einem Reaktor durchgeführt werden. Der Reaktor kann gegebenenfalls mit Zwischenkühlung betrieben werden. Dabei erfolgt die Umsetzung im rückvermischten Reaktorsystem. Im rückvermischten Reaktor wird der Umlaufstrom gekühlt. Eine Aufteilung in mehrere Betten, gegebenenfalls auch mit Zwischenkühlung, kann ebenfalls in dem einen Reaktor realisiert werden.

Nach dem Austritt aus dem Reaktor folgt mindestens eine Destillationsstufe. Diese können parallel oder in Reihe betrieben werden. Bevorzugt sind sie in Reihe geschaltet.

Figur 1 zeigt eine Ausführungsform des erfindungsgemäßen Verfahrens mit einem Reaktor (1) und einer Trennkolonne (8). Über drei Zuführungen werden die drei Edukte Isoprenol (A), Aldehyd (B) und Wasser (C) in den Reaktor (1) eingeleitet. Über eine Leitung und die Pumpe (5) wird ein Austrag aus dem Reaktor (1) entnommen, der in zwei Teilströme geteilt wird. Über den Kühler (2) wird ein Rückführstrom (D) mit den Edukten (A), (B) und (C) zusammen in den Hauptreaktor (1) geführt. Ein Feed (E) wird der Trennkolonne (8) zugeführt. Das Edukt (G) wird als Austrag am Boden der Trennkolonne (8) entnommen und gegebenenfalls einer Aufarbeitung zugeführt. Am Kopf wird der Trennkolonne (8) das Kopfprodukt (F) entnommen und zumindest teilweise in den Reaktor (1) zurückgeführt.

Der Reaktor (1) wird bevorzugt als Festbettreaktor ausgeführt. Dabei wird er in Schlaufenfahrweise betrieben, wohingegen die Trennkolonne (8) im geraden Durchgang betrieben wird. In der in Figur 1 dargestellten Anordnung sind der Reaktor (1) und die Trennkolonne (8) so in Reihe geschaltet, dass über die Rückvermischung im Reaktor (1) die Einstellung eines Temperaturprofils über dem Katalysatorbett erfolgen kann. Dadurch kann ein starker Temperaturanstieg zu Beginn der Reaktion verhindert werden.

Die folgenden Beispiele dienen der Verdeutlichung der Erfindung, ohne sie in irgendeiner Weise zu beschränken.

### BEISPIELE

### Beispiel 1 (kontinuierlich betriebener Prozess mit Teilumsatz und Rückführung)

Es wurde eine Apparatur, bestehend aus einem Reaktor und einer Laborkolonne verwendet. Als Reaktor wurde ein Doppelmantelreaktor aus RA4 ohne Heizmedium für eine adiabatische Fahrweise mit einer Länge von 150 cm und einem Innendurchmesser von 2,6 cm verwendet.

Der Reaktor wurde mit 230 g (305 ml) des Kationenaustauschers gefüllt. Der Kationenaustauscher wurde vor dem Einsatz zunächst mehrmals mit Wasser, dann einmal mit Methanol und abschließend mit Wasser Methanol-frei gewaschen. Anschließend wurde der Reaktor durch Zufahren einer Mischung aus Pyranol : Wasser im MassenVerhältnis von 95 : 5 konditioniert. Der Hauptreaktor wurde anschließend rückvermischt mit einem Umwälzstrom von 2000 g/h betrieben, wobei der rückgeführte Strom vor Wiedereintritt in den Hauptreaktor auf eine Temperatur von 25 °C abgekühlt wurde. Nach Konditionierung des Kationenaustauschers auf das genannte Pyranol-WasserGemisch wurde ein Gemisch aus Isovaleraldehyd : Isoprenol : Wasser im MassenVerhältnis von 45 : 50 : 5 bei 25 °C und einem Gesamtmengenstrom von 100 g/h zugefahren. Man erhielt ein Rohprodukt mit einer Selektivität von 78,5 % bezüglich Isovaleraldehyd mit der folgenden Zusammensetzung:
Isovaleraldehyd: 10,25 GC-Gew.-%,
Isoprenol: 13,95 GC-Gew.-%,
Dihydropyran-Isomere: 6,98 GC-Gew.-%,
1,3-Dioxan: 3,18 GC-Gew.-%,
Acetal: 0,63 GC-Gew.-%,
trans-Pyranol: 14,60 GC-Gew.-%,
cis-Pyranol: 40,07 GC-Gew.-%,
Wasser: 6,8 Gew.-% (nach Karl Fischer).

Die Reaktionsmischung wurde anschließend der simultan laufenden, kontinuierlichen destillativen Trennung unterzogen, wobei das Kopfprodukt, bestehend aus Isovaleraldehyd, Isoprenol und Wasser, dem Zulauf des Hauptreaktors zugemischt und somit zurückgeführt wurde. Gleichzeitig wurde der Zulauf der Edukte zum Hauptstrom um die entsprechende Menge reduziert.

## Patentansprüche

1. Verfahren zur Herstellung von 2-substituierten 4-Hydroxy-4-methyl-tetrahydropyranen der allgemeinen Formel (I) 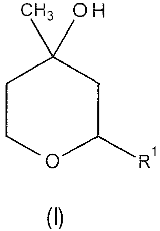 worin
R¹ für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
umfassend eine Umsetzung von 3-Methylbut-3-en-1-ol der Formel (III) 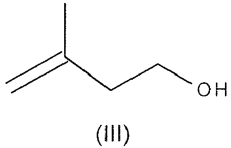 mit einem Aldehyd der Formel (IV)
R¹-CHO (IV)
wobei R¹ in der Formel (IV) die zuvor angegebene Bedeutung hat,
in Gegenwart eines sauren Katalysators, **dadurch gekennzeichnet, dass** die Umsetzung in einem Reaktor mit wenigstens einer nachgeschalteten Trennkolonne erfolgt, wobei ein Teilstrom des Austrags aus dem Reaktor abgezogen und über einen externen Kreislauf in den Reaktor zurückgeführt wird, und wobei dem Teilstrom des Austrags aus dem Reaktor vor der Rückführung in den Reaktor Wärme entzogen wird.

2. Verfahren nach Anspruch 1, **dadurch gekennzeichnet, dass** die Umsetzung kontinuierlich erfolgt.

3. Verfahren nach einem der Ansprüche 1 oder 2, **dadurch gekennzeichnet, dass** die Umsetzung in Gegenwart eines Lösungsmittels, insbesondere in Gegenwart von Wasser erfolgt.

4. Verfahren nach einem der Ansprüche 1 bis 3, **dadurch gekennzeichnet, dass** dem Austrag aus dem Reaktor vor der Zuführung in die Trennkolonne zusätzlich Wärme zugeführt wird.

5. Verfahren nach einem der Ansprüche 1 bis 4, **dadurch gekennzeichnet, dass** dem Austrag aus dem Kopf der wenigstens einen Trennkolonne zumindest ein Teilstrom entnommen und über eine externe Rückführung in den Reaktor zurückgeführt wird.

6. Verfahren nach einem der Ansprüche 1 bis 5, **dadurch gekennzeichnet, dass** die Umsetzung im Reaktor adiabatisch durchgeführt wird, wobei unter adiabatischer Reaktionsführung eine Vorgehensweise im technischen Sinne verstanden wird, bei der die bei der Reaktion freiwerdende Wärmemenge von der Reaktionsmischung im Reaktor aufgenommen und keine Kühlung durch Kühlvorrichtungen erfolgt, wodurch die Reaktionswärme weitestgehend mit dem Reaktionsgemisch aus dem Reaktor abgeführt und ein Restanteil durch natürliche Wärmeleitung bzw. -strahlung vom Reaktor an die Umgebung abgegeben wird.

7. Verfahren nach einem der Ansprüche 1 bis 6, **dadurch gekennzeichnet, dass** der verwendete Reaktor weitgehend isotherm betrieben wird, wobei weitgehend isotherm bedeutet, dass das Temperaturintervall ΔT im Reaktor kleiner als die adiabatische Temperaturerhöhung ist.

8. Verfahren nach Anspruch 7, **dadurch gekennzeichnet, dass** der verwendete Reaktor einen intern angeordneten Wärmeübertrager umfasst.

9. Verfahren nach einem der vorhergehenden Ansprüche, **dadurch gekennzeichnet, dass** der verwendete Reaktor ein Festbettreaktor ist.

10. Verfahren nach einem der vorhergehenden Ansprüche, **dadurch gekennzeichnet, dass** der Rest R¹ für Isobutyl oder Phenyl steht.

11. Verfahren nach einem der vorhergehenden Ansprüche, **dadurch gekennzeichnet, dass** die Umsetzung in Gegenwart eines sauren Katalysators erfolgt, der vorzugsweise ausgewählt ist unter Salzsäure, Schwefelsäure, Methansulfonsäure, p-Toluolsulfonsäure und stark sauren Kationenaustauschern.

12. Verfahren nach Anspruch 11, **dadurch gekennzeichnet, dass** die Umsetzung in Gegenwart eines stark sauren Kationenaustauschers durchgeführt wird.

13. Verfahren nach einem der vorhergehenden Ansprüche, **dadurch gekennzeichnet dass** der Alkohol der Formel (III) und der Aldehyd der Formel (IV) in einem molaren Verhältnis im Bereich von 0,7 : 1 bis 2 : 1 eingesetzt werden.

14. Verfahren nach einem der vorhergehenden Ansprüche, **dadurch gekennzeichnet dass** der Alkohol der Formel (III) und der Aldehyd der Formel (IV) in Gegenwart von 3 Gew.-% bis 15 Gew.-% Wasser, bevorzugt von 5 Gew.-% bis 12 Gew.-%, bezogen auf die Menge des Reaktionsgemisches bestehend aus den Komponenten der Formeln (III) und (IV) sowie Wasser, eingesetzt werden.

15. Verfahren nach einem der vorhergehenden Ansprüche, **dadurch gekennzeichnet dass** die Umsetzung bei einer Temperatur im Bereich von 0 °C bis 70 °C, bevorzugt im Bereich von 20 °C bis 70 °C, besonders bevorzugt im Bereich von 20 °C bis 60 °C durchgeführt wird.

## Claims

1. A process for the preparation of 2-substituted 4-hydroxy-4-methyltetrahydropyrans of the general formula (I) 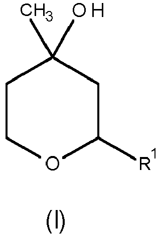 in which
R¹ is a straight-chain or branched C₁-C₁₂-alkyl, straight-chain or branched C₂-C₁₂-alkenyl, unsubstituted or C₁-C₁₂₋alkyl and/or C₁-C₁₂-alkoxy substituted cycloalkyl having in total 3 to 20 carbon atoms or unsubstituted or C₁-C₁₂₋alkyl and/or C₁-C₁₂₋alkoxy substituted aryl having in total 6 to 20 carbon atoms,
comprising a reaction of 3-methylbut-3-en-1-ol of the formula (III) 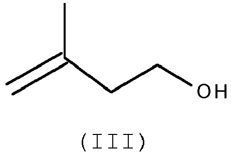 with an aldehyde of the formula (IV)
R¹-CHO (IV)
where R¹ in the formula (IV) has the meaning given above,
in the presence of an acidic catalyst, wherein the reaction takes place in a reactor with at least one downstream separating column, where a part stream of the discharge from the reactor is stripped off and returned to the reactor via an external circuit, and where heat is removed from the part stream of the discharge from the reactor prior to returning it to the reactor.

2. The process according to claim 1, wherein the reaction takes place continuously.

3. The process according to either of claims 1 and 2, wherein the reaction takes place in the presence of a solvent, in particular in the presence of water.

4. The process according to any one of claims 1 to 3, wherein heat is additionally supplied to the discharge from the reactor before feeding it to the separating column

5. The process according to any one of claims 1 to 4, wherein at least one part stream is removed from the discharge from the top of the at least one separating column and returned to the reactor via an external recirculation.

6. The process according to any one of claims 1 to 5, wherein the reaction in the reactor is carried out adiabatically, where adiabatic reaction implementation is understood in the technical sense as meaning a procedure in which the amount of heat that is released during the reaction is absorbed by the reaction mixture in the reactor and no cooling takes place by means of cooling devices, so that the heat of reaction is substantially removed from the reactor with the reaction mixture and a residual amount is released into the surroundings as a result of natural heat conduction and/or radiation from the reactor.

7. The process according to any one of claims 1 to 6, wherein the reactor used is operated largely isothermally, where largely isothermally is understood as meaning that the temperature interval Δ T in the reactor is smaller than the adiabatic temperature increase.

8. The process according to claim 7, wherein the reactor used comprises an internally arranged heat exchanger.

9. The process according to any one of the preceding claims, wherein the reactor used is a fixed-bed reactor.

10. The process according to any one of the preceding claims, wherein the radical R¹ is isobutyl or phenyl.

11. The process according to any one of the preceding claims, wherein the reaction takes place in the presence of an acidic catalyst which is preferably selected from hydrochloric acid, sulfuric acid, methanesulfonic acid, p-toluenesulfonic acid and strongly acidic cation exchangers.

12. The process according to claim 11, wherein the reaction is carried out in the presence of a strongly acidic cation exchanger.

13. The process according to any one of the preceding claims, wherein the alcohol of the formula (III) and the aldehyde of the formula (IV) are used in a molar ratio in the range from 0.7:1 to 2:1.

14. The process according to any one of the preceding claims, wherein the alcohol of the formula (III) and the aldehyde of the formula (IV) are used in the presence of 3% by weight to 15% by weight of water, preferably from 5% by weight to 12% by weight, based on the amount of the reaction mixture consisting of the components of the formulae (III) and (IV) and also water.

15. The process according to any one of the preceding claims, wherein the reaction is carried out at a temperature in the range from 0°C to 70°C, preferably in the range from 20°C to 70°C, particularly preferably in the range from 20°C to 60°C.

## Revendications

1. Procédé de fabrication de 4-hydroxy-4-méthyl-tétrahydropyranes 2-substitués de formule générale (I) 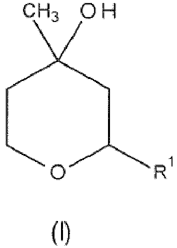 dans laquelle
R¹ représente alkyle en C₁-C₁₂ linéaire ou ramifié, alcényle en C₂-C₁₂ linéaire ou ramifié, cycloalkyle non substitué ou substitué avec alkyle en C₁-C₁₂ et/ou alcoxy en C₁-C₁₂, contenant au total 3 à 20 atomes de carbone, ou aryle non substitué ou substitué avec alkyle en C₁-C₁₂ et/ou alcoxy en C₁-C₁₂, contenant au total 6 à 20 atomes de carbone,
comprenant une mise en réaction de 3-méthylbut-3-én-1-ol de formule (III) 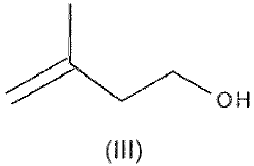 avec un aldéhyde de formule (IV)
R¹-CHO (IV)
R¹ dans la formule (IV) ayant la signification indiquée précédemment,
en présence d'un catalyseur acide, **caractérisé en ce que** la mise en réaction a lieu dans un réacteur comprenant au moins une colonne de séparation en aval, dans laquelle un courant partiel de la sortie du réacteur est soutiré et recyclé par un circuit externe dans le réacteur et dans laquelle de la chaleur est extraite du courant partiel de la sortie du réacteur avant le recyclage dans le réacteur.

2. Procédé selon la revendication 1, **caractérisé en ce que** la réaction a lieu en continu.

3. Procédé selon l'une quelconque des revendications 1 ou 2, **caractérisé en ce que** la réaction a lieu en présence d'un solvant, notamment en présence d'eau.

4. Procédé selon l'une quelconque des revendications 1 à 3, **caractérisé en ce que** de la chaleur est en outre introduite dans la sortie du réacteur avant l'introduction dans la colonne de séparation.

5. Procédé selon l'une quelconque des revendications 1 à 4, **caractérisé en ce qu'**au moins un courant partiel est soutiré de la sortie de la tête de ladite au moins une colonne de séparation et recyclé dans le réacteur par une conduite de recyclage externe.

6. Procédé selon l'une quelconque des revendications 1 à 5, **caractérisé en ce que** la réaction dans le réacteur est réalisée sous forme adiabatique, une réalisation adiabatique de la réaction étant comprise au sens technique comme une procédure selon laquelle la quantité de chaleur libérée lors de la réaction est absorbée par le mélange réactionnel dans le réacteur et aucun refroidissement n'a lieu par des dispositifs de refroidissement, la chaleur de réaction étant ainsi très largement déchargée du réacteur avec le mélange réactionnel et une fraction résiduelle étant émise dans l'environnement par conduction ou rayonnement de chaleur naturel depuis le réacteur.

7. Procédé selon l'une quelconque des revendications 1 à 6, **caractérisé en ce que** le réacteur utilisé est exploité essentiellement sous forme isotherme, essentiellement sous forme isotherme signifiant que l'intervalle de température ΔT dans le réacteur est inférieur à l'élévation de température adiabatique.

8. Procédé selon la revendication 7, **caractérisé en ce que** le réacteur utilisé comprend un échangeur de chaleur agencé en interne.

9. Procédé selon l'une quelconque des revendications précédentes, **caractérisé en ce que** le réacteur utilisé est un réacteur à lit fixe.

10. Procédé selon l'une quelconque des revendications précédentes, **caractérisé en ce que** le radical R¹ représente isobutyle ou phényle.

11. Procédé selon l'une quelconque des revendications précédentes, **caractérisé en ce que** la réaction a lieu en présence d'un catalyseur acide, qui est de préférence choisi parmi l'acide chlorhydrique, l'acide sulfurique, l'acide méthane-sulfonique, l'acide p-toluène-sulfonique et les échangeurs de cations fortement acides.

12. Procédé selon la revendication 11, **caractérisé en ce que** la réaction est réalisée en présence d'un échangeur de cations fortement acide.

13. Procédé selon l'une quelconque des revendications précédentes, **caractérisé en ce que** l'alcool de formule (III) et l'aldéhyde de formule (IV) sont utilisés en un rapport molaire dans la plage allant de 0,7:1 à 2:1.

14. Procédé selon l'une quelconque des revendications précédentes, **caractérisé en ce que** l'alcool de formule (III) et l'aldéhyde de formule (IV) sont utilisés en présence de 3 % en poids à 15 % en poids d'eau, de préférence de 5 % en poids à 12 % en poids, par rapport à la quantité du mélange réactionnel constitué par les composants de formule (III) et (IV), ainsi que de l'eau.

15. Procédé selon l'une quelconque des revendications précédentes, **caractérisé en ce que** la réaction est réalisée à une température dans la plage allant de 0 °C à 70 °C, de préférence dans la plage allant de 20 °C à 70 °C, de manière particulièrement préférée dans la plage allant de 20 °C à 60 °C.
